# EUROPEAN PATENT APPLICATION

(11) **EP 1 992 704 A1**
(43) Date of publication of application: **19.11.2008**
(21) Application number: 07817197.2
(22) Date of filing: 02.11.2007
(51) Int. Cl.: C12Q 1/68

(54) **TAQMAN MGB PROBE FOR DETECTING MATERNAL INHERITED MITOCHONDRIAL GENETIC DEAFNESS C1494T MUTATION AND ITS USAGE**

(30) Priority: 26.12.2006 CN 200610169639
(71) Applicant: Jin, Zhengce, Beijing 100027 (CN)
(72) Inventor: DAI, Pu, Beijing 100027 (CN); YUAN, Yongyi, Beijing 100027 (CN); HAN, Dongyi, Beijing 100027 (CN)
(74) Representative: Ziebig, Marlene
(86) International application number: PCT/CN2007/071006
(87) International publication number: WO 2008/077330

(57) **Abstract**

The present invention relates to a real time quantitative TaqMan MGB probe useful for detecting the mitochondrial gene C1494T mutation associated with maternally inherited deafness and the use thereof. The present invention design a Taqman mutant probe and a wild type MGB probe, and a pair of primers. A maternally inherited deafness associated with mitochondrial gene C1494T mutation can be diagnosed by the method of real time quantitative Taqman MGB probe. This method is characteristic of easily operating, fast, high specificity, high sensitivity, and the interpretation of the result is intuitionistic, accurate and reliable. It's suitable for large scale screen and preventive examination of mitochondrial gene C1494T mutation associated with maternally inherited deafness.

## Description

### Technical Field

The present invention relates to the field of genetic engineering, particularly, relates to a probe useful for diagnosing a maternally inherited deafness associated with mitochondrial gene mutation and the use thereof. More particularly, relates to real time quantitative TaqMan MGB probe useful for detecting the mitochondrial gene C1494T mutation associated with maternally inherited deafness and the use thereof. The present invention further relates to the use of the MGB probe and related product in the preparation of kit or identical product useful for diagnosing a maternally inherited deafness associated with mitochondrial gene mutation.

### Background of the invention

Aminoglycoside antibiotics (streptomycin, gentamycin, kanamycin, tobramycin and micronomicin, etc.) were used in the controlling of infection of gram negative and gram positive bacterials clinically for their broad spectrum antibacterial function and their cheap price. However, this kind of antibiotics possess fearful ototoxic side effect, which will lead to irreversible hearing loss. Researches in the recent decade suggested that, some deaf patients induced by aminoglycoside antibiotics are associated with maternally inherited family history, and are associated with the homozygous C→T point mutation at position 1494 (hereinafter referred as C1494T mutation) in mitochondrial DNA 12S rRNA gene (hereinafter referred as mitochondrial gene, or mtDNA) (Zhao H et al, American Journal of Human Genetics, 2004, 74: 139-152; Wang Q et al, Biochem Biophys Res Commun. 2006, 340:583-588). Administration of a single dose of aminoglycoside is able to induce a severe hearing loss in the individuals with this mutation. Based on currently available findings, almost all of the individuals with this mutation have severe or profound hearing loss after administration of various dose of aminoglycoside antibiotics. It is a significant factor causing acquired deafness. This kind of deafness is a preventable disease in that the defined gene change and the inducing factor associated with it has been confirmed.

The mitochondrial gene C1494T mutation (mtDNA C1494T mutation) is a type of mitochondrial gene mutation which have been identified to be capable of leading to ototoxic deafness after administration of a single dose of aminoglycoside drug. The mitochondrial gene C1494T mutation occurred in maternally inherited deafness have been found in four pedigrees in China , there are 2 to 28 individuals with hearing loss in each pedigree. There are a number of similar pedigrees which have not been found and reported and this mutation account for certain proportion in the pathogenic mechanism of sporatic deafness. So the prevention of this kind of deafness becomes more important. The critical practice of preventing this disease is to screen the individuals with mtDNA C1494T mutation, and prohibit the individuals from exposing to aminoglycoside antibiotics so as to prevent the occurrence of severe ototoxic reaction.

Since the correlation between mtDNA C1494T and deafness was confirmed in 2004, the detection method of this mutation is direct sequencing. Sequencing is an available approach for evaluating gene mutation, however it needs expensive equipment and complex operation. It is a time and labor consuming method. In order to overcome the drawbacks mentioned above, we designed a novel testing method which meet the needs for wide spread screening of mtDNA C1494T mutation. It is a simple, fast, accurate, less pollution and inexpensive method.

### Summary of the invention

The objects of the invention was achieved through the following principles:
In order to detect the C1494T mutation in the maternally inherited mitochondrial gene sequence (SEQ ID NO:5), a TaqMan MGB probe( probe sequence including the region from 1482bp to 1508bp) was designed which including the position 1494bp of said gene sequence. The 5' end of the MGB probe fragment was labeled with fluorescent reporter group, and the 3' end was labeled with non-fluorescent quenching group MGB, wherein: in the case where the C base at position 1494bp of the MGB probe is substituted with T, it is referred to as mutant real time quantitative MGB probe (or mutant MGB probe, mutant Taqman MGB probe) ; in the case where the C base at position 1494bp of the MGB probe keep unchanged, it is referred to as wild type real time quantitative MGB probe (or wild type MGB probe, wild type Taqman MGB probe) ;

As the 5' end fluorescent reporter group of the probe of the mutant and wild type MGB are different, fluorescence of different wavelength can be recorded in a real time quantitative fluorescence PCR apparatus independently, they are displayed in different colors or different labels through analysis software . Therefore, the template with C1494T mutation capable of matching with the mutant MGB probe completely, however the wild type template does not. The wild type MGB probe can not matched with the template with C1494T mutation completely, it is capable of matching with the wild type template completely. Furthermore, the templates conjunct with different MGB probes will emit fluorescence of different wavelength.

In real time quantitative PCR amplification system, a forward primer and a reverse primer (designed with the aid of any available primer designing software) useful for amplifying the gene sequence associated with maternally inherited mitochondrial deafness as set forth in SEQ ID NO:5 were added, a mutant real time quantitative MGB probe was added as well. If desired, a wild type real time quantitative MGB probe was further included.

Since the MGB probe is rather short (about 12-25bp in length) , the absorbed energy of 5' end fluorescent reporter group is able to transfer to the adjacent 3' end non-fluorescent quenching group, and the 3' end non-fluorescent quenching group is able to inhibit the fluorescence emmission of 5' end fluorescent reporter group. Therefore, in the case where the MGB probe is intact, that is the 5' end fluorescent reporter group is under the protection of the 3'non-fluorescent quenching group, no fluorescence emitting from the fluorescence group at the 5' end of MGB probe can be detected.

In one embodiment, the primer and the mutant MGB probe and the wild type MGB probe were added into the real time quantitative PCR reaction system simultaneously. Both primer and MGB probe will conjunct with the template when the denatured template in this system annealing at low temperature. While extending to the point where the MGB probe joint along with the template mediated by the primer, the fluorescent reporter group joint at the 5' end of the MGB probe was cut out from the MGB probe by the 5'-3' exonuclease activity of Taq enzyme (the activity is double chain specific, the free single chain MGB probe keep intact) , and then released into the reaction system. It departed from the shield of the 3' end non-fluorescent quenching group and emitted fluorescence signal under light stimulus. One fluorescence molecule will form when one new DNA chain amplifies. The accumulation of fluorescence signals and the production of PCR products are performed simultaneously. In other words, the strength of fluorescence signals is the linear function of the production of PCR products. In the case where the template is C1494T mutant, the junction between mutant MGB probe and it matched completely. The 5' end fluorescent reporter groups were removed by Taq enzyme continually during PCR reaction, departing from the shield of the 3' end non-fluorescent quenching groups, and the strength of fluorescence increased with the increasing of PCR products. The wild type MGB probe can not conjunct with it as there is a difference of one base. And only the fluorescence signal emitting from the mutant MGB probe can be detected. In the case where the template is C1494 C wild type, the conjunction between the wild type MGB probe and the template matched perfectly. The mutant MGB probe can not conjunct with it as there is a difference of one base, only the fluorescence signal emitting from the wild type MGB probe can be detected. Since the 5' end of the mutant type and wild type MGB probe joined with different fluorescent reporter groups respectively, the templates corresponding to different fluorescence can be referred to each other based on the different colors of different fluorescence. Thereby whether a mitochondrial gene associated with maternally inherited deafness exist in a sample or not can be easily determined.

In another embodiment, only the mutant MGB probe was added into the real time quantitative PCR reaction system. In the case where the template is mutant type, the fluorescence signal emitting from the mutant MGB probe can be detected. Thereby the sample containing the mitochondrial gene C 1494T mutation associated with maternally inherited deafness and the patient suffering from a maternally inherited deafness associated with mitochondrial gene mutation can be determined. In the case where the template is wild type, the fluorescence signal emitting from the mutant MGB probe can not be detected. Thereby the sample do not containing the mitochondrial gene C1494T mutation associated with maternally inherited deafness and the patient without a maternally inherited deafness associated with mitochondrial gene mutation can be determined.

That is to say, theoretically, the test can be performed in the case where only the mutant MGB probe corresponding to the C1494T mutant template was added into the reaction system. In the case where the system includes the wild type MGB probe corresponding to the C1494C wild type template as well, dual fail-safe effect can be achieved through reverse correction. The results obtained is more accurate and reliable.

Therefore, based upon the inventive concepts, in one aspect, the present invention provides a real time quantitative MGB probe useful for detecting the mitochondrial gene C1494T mutation associated with maternally inherited deafness, wherein the 5' end of the MGB probe is located in the region between 1482-1487 bp in the mitochondrial gene sequence as set forth in SEQ ID No:5 associated maternally inherited deafness, the 3' end is in the region of 1503-1508 bp in the mitochondrial gene sequence as set forth in SEQ ID NO:5 associated maternally inherited deafness. And the 5' end of this MGB probe fragment was labeled with fluorescent reporter group, the 3' end was labeled with non-fluorescent quenching group MGB. The C base at position 1494bp was substituted with T, thereby was referred to as mutant real time quantitative MGB probe.

In addition, it's known that the TaqMan probe can be classified into two types based on the difference between the 3' end labled quenching groups: Common TaqMan probe and TaqMan MGB probe. The quenching group of common TaqMan probe is fluorescent quenching group, and the quenching group of MGB probe is non-fluorescent quenching group. It does not generate fluorescence per se, thereby the strength of background signal can be reduced significantly. Meanwhile as the probe is joined with MGB (Minor Groove Binder) modifying group, the Tm of the probe can be increased about 10°C. In order to obtain similar Tm value, the designed MGB probe is shorter than common TaqMan probe. This is helpful to reduce cost of synthesis and increase the success rate of probe design. In the case where the DNA base composition of the template is non ideal, the design of a short probe is easier than a longer one. Based on the features of the base composition at and around the mtDNA 1494 site, and extensive experiments, the 5' end of TaqMan MGB probe used in the present invention is preferably located in the region between 1485-1488bp of SEQ ID NO:5, the 3' end is locate in the region between 1503-1505bp. More preferably, the 5' end of TaqMan MGB is located in the region between 1487-1488bp, the 3' end is located at 1503bp.

In one particular embodiment, the nucleotide sequence of the mutant MGB probe is located in the region between 1487-1503bp (the nucleotide sequence as set forth in SEQ ID NO:1) of the gene sequence associated with maternally inherited mitochondrial deafness as set forth in SEQ ID NO:5, and the 5' end fluorescent reporter group is FAM fluorescent reporter group, the 3' end group is non-fluorescent quenching group MGB.

In another aspect, the present invention provides a wild type MGB probe used in conjunction with the real time quantitative MGB probe. The wild type real time MGB probe is same as the mutant real time MGB probe, except that the base at position 1494bp still be C, the 5' end fluorescent reporter group is different from the 5' end fluorescent reporter group of the mutant MGB probe, and the starting point of the probe is locate at 1488bp rather than 1487bp.

In one particular embodiment, the nucleotide sequence of the wild type MGB probe is located in the region between 1488-1503bp (the nucleotide sequence as set forth in SEQ ID NO:2) of the gene sequence associated with maternally inherited mitochondrial deafness as set forth in SEQ ID NO:5, and the 5' end fluorescent reporter group is HEX fluorescent reporter group, the 3' end is non-fluorescent quenching group MGB.

It should be noted that, the 5' end fluorescent reporter group and the corresponding 3' end non-fluorescent quenching group may be any available fluorescent reporter group or non-fluorescent quenching group known in this field. With respect to the skilled technician in this field, the choice of said fluorescent reporter group and the corresponding non-fluorescent quenching group is a routine practice. For example, the fluorescence dye FAM and HEX can be replaced by other fluorescence group or dye, such as VIC, JOE etc.

In another aspect, the present invention provide a forward primer and a reverse primer used in conjunction with the real time quantitative MGB probe in PCR reaction. The forward primer and the reverse primer were used in the amplification of the gene sequence associated with maternally inherited mitochondrial deafness as set forth in SEQ ID NO:5 which including the C1494T mutation. Wherein the 5' end of forward primer (about 15-24bp in length) is located in the region between 1-1464bp of SEQ ID NO:5 (upstream of C1494T) , the 3'end of the reverse primer (about 15-24bp in length) is located in the region between 1615bp-16568bp of SEQ ID NO:5 (downstream of C1494T). Preferably, the 5'end of the forward primer (about 15-24bp in length) is located in the region between 1000-1464bp of SEQ ID NO:5 (upstream of C1494T) , the 3'end of the reverse primer (about 15-24bp in length) is located in the region between 1615bp-2000bp of SEQ ID NO:5 (downstream of C1494T) . More preferably, the 5' end of the forward primer (about 15-24bp in length) is located in the region between 1400-1464bp of SEQ ID NO:5 (upstream of C1494T) , the 3' end of the reverse primer (about 15-24bp in length) is located in the region between 1615bp-1700bp of SEQ ID NO:5 (downstream of C1494T) . Most preferably, the 5' end of the forward primer (about 15-24bp in length) is located at 1464bp of SEQ ID NO:5 (upstream of C1494T) , the 3' end of the reverse primer (about 15-24bp in length) is located at 1615bp of SEQ ID NO:5 (downstream of C1494T).

In one particular embodiment, the forward primer is the nucleotide sequence between 1464-1481bp of the gene sequence associated with maternally inherited mitochondrial deafness as set forth in SEQ ID NO:5, the sequence thereof is set forth in SEQIDNO:3 in sequence list; the reverse primer is the nucleotide sequence between 1592-1615 bp as set forth in SEQ ID NO:5, the sequence thereof is set forth in SEQIDNO:4 in sequence list.

In one particular embodiment, the primer can be designed with the aid of any available biosoftware. Preferably, the Genetool Lite program (a software available from Biotools company, USA) was used in the designing of primer.

In another aspect, the present invention provides a use of said MGB probe in the preparation of a kit useful for diagnosing maternal mitochondrial deafness.

In one particular embodiment, the primer and Taqman MGB probe of this invention were used in a kit useful for diagnosing a maternally inherited deafness caused by mitochondrial gene C1494T mutation, useful for diagnosing a maternally inherited deafness associated with mitochondrial gene mutation, the kit comprises:
1. reagents useful for isolating blood DNA;
2. Mixture of reagents useful for PCR amplification reaction, preferably the mixture of Hotstar Mastermix;
3. The forward primer and the reverse primer useful for amplifying the gene sequence associated with maternally inherited mitochondrial deafness as set forth in SEQ ID NO:5 comprising C1494C site or C1494T mutation site (preferably mixed at a ratio of 1: 1);
4. The real time quantitative mutant Taqman MGB probe mentioned above;
5. If desired, Operation instruction.

In one particular embodiment, said kit further comprises the wild type real time quantitative Taqman MGB probe mentioned above.

In another particular embodiment, the structure of the forward primer and the reverse primer included in said kit is as described above. The forward primer is preferably selected from the nucleotide sequences as set forth in SEQ ID NO:3, the reverse primer is preferably selected from the nucleotide sequences as set forth in SEQ ID NO:4.

In another particular embodiment, the nucleotide sequence of mutant MGB probe is preferably selected from the nucleotide sequences as set forth in SEQ ID NO:1. The fluorescent reporter group at the 5' end is FAM fluorescent reporter group, the non-fluorescent quenching group used to label the 3' end is non-fluorescent quenching group MGB.

In another particular embodiment, the nucleotide sequence of wild type MGB probe is preferably selected from the nucleotide sequences as set forth in SEQ ID NO:2, The fluorescent reporter group at the 5' end is HEX fluorescent reporter group, the non-fluorescent quenching group used to label the 3' end is non-fluorescent quenching group MGB.

In another particular embodiment, The reagents included in said kit useful for isolating blood DNA is solution I which is useful for isolating DNA from plantar blood. The principal constituents of it is Chelex (A product available from ABI company). Alternatively, the reagent used for isolating blood DNA is a reagent useful for isolating DNA from peripheral blood, used in conjunction with commercially available corresponding product. It should be noted that, the term "kit" including another designation rather than kit, which is similar to the kit in structure or composition and biological functions.

In another particular embodiment, the present invention provides a use of said MGB probe or kit in the in vitro detecting of the mitochondrial gene C1494T mutation associated with maternally inherited deafness. In another particular embodiment, the present invention further provides a use of said MGB probe or kit in the preparation of products (including reagents, kits etc.) useful for diagnosing maternally inherited mitochondrial deafness (that is to detect the mitochondrial gene C 1494T mutation associated with maternally inherited deafness) .

The method for detecting the mitochondrial gene C1494T mutation associated with maternally inherited deafness have the following advantages as compared with the previous method (such as Hae III restriction analysis) :
1. The present method for detecting mitochondrial gene C1494T mutation is more efficient than the direct sequencing method in both operational procedure and reaction time: the present method is able to determine whether a mtDNA C1494T mutation exist or not through only one PCR cycle. The steps of agarose gel electrophoresis analysis, the purification of PCR products, sequencing and the purification of products after the PCR reaction were omitted as compared with sequencing method. Thereby the risk of pollution from the processing post the PCR reaction is reduced, and the original operation time (12 hours ) is reduced to 1.5 hour.
2. The design of two strand of MGB probe is corresponding to the C1494T mutant and C1494C wild type respectively. In the case where they are included in the same system, each MGB probe only conjunction with the template which matched with it completely. The binding between them is of highly specificity. With respect to a template, in the case where the fluorescence (such as FAM fluorescence) at the 5' end of mutant MGB probe is detected, and in the case where no fluorescence (such as HEX fluorescence) at the 5' end of wild type MGB probe is detected, it can be determined as C1494T mutant. On the contrary, it can be determined as C1494C wild type. Theoretically, the diagnosis can be performed in the case where only the mutant MGB probe corresponding to the C1494T mutant template is included in a reaction system. In the case where the system includes both type of MGB probes, dual fail-safe effect can be achieved through reverse correction and the results obtained is more accurate and reliable. In addition, the high specificity of MGB probe technique and the sensitivity of spectral technique endue the present invention an excellent repeatability, high specificity, high sensitivity and easy manipulation.
3. The interpretation of the results of this invention is performed with the aid of analysis software of the real time quantitative PCR apparatus. The procedure is automatized and artificial error can be reduced.
4. The cost of the detection of C1494T mutation performed with the kit or related products provided by the invention is lower than that of conventional method.

In this way, the present MGB probe and the kit thereof meet the requirements of related technical field. It has the advantages of operating easily and the interpretation of the result is characteristic of intuition, high specificity, high sensitivity and low cost. It provides advantageous conditions for screening the mitochondrial gene C1494T mutation associated with maternally inherited deafness and preventive examination before administration of aminoglycoside antibiotics nationally. Thereby the possibility of antibiotics-induced deafness occurred in the sensitive individuals caused by aminoglycoside antibiotics can be reduced radically.

### The description of the drawings

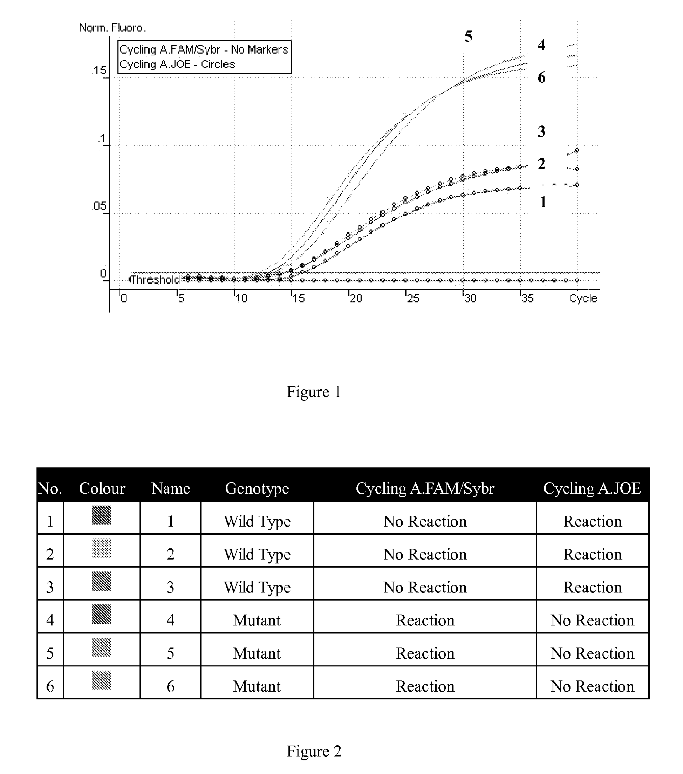

Figure 1 is a graphic of part results from the real time quantitative PCR analysis of 72 patients with different genotype (Allelic data for Cycling A.FAM/Sybr, Cycling A.JOE).
Figure 2 is a legend of part results from the real time quantitative PCR analysis of 72 patients with different genotype.

### Embodiments

The detection of the C1494T mutation in mitochondrial gene associated with maternally inherited deafness

### 1. Test sample

Seventy-two sensorineural deaf patients were selected from the Molecular diagnostic lab for deafness of PLA General Hospital. DNA were isolated from peripheral whole blood of selected individuals (Yuan huijun etal., Chinese Journal of Otorhinolaryngology, 1998, 33 (2) :67-70; Li weimin etal., Journal of Clinical Otorhinolaryngology, 2001, 15(supplement): 53-58) and used as test sample.

### 2. Designing of MGB probe and primer

The primer and Taqman MGB probe sequences were designed based on the public mitochondrial gene sequence (Human mitochondrial genome sequence NC_001807.4 or NT_006713.14 etc., or SEQ ID NO:5 from Cambridge Sequence or NCBI) with the aids of Genetool Lite program, wherein:
The nucleotide sequence of mutant MGB probe (T1) locates in the region between 1487-1503 bp (the nucleotide sequence as set forth in SEQ ID NO:1) of the gene sequence associated with maternally inherited mitochondrial deafness as set forth in SEQ ID NO:5. The fluorescent reporter group is FAM fluorescent reporter group, and the non-fluorescent quenching group is MGB. The formula of MGB probe is as follows:

### 5' FAM-CCGTCACTCTCCTCAAG-MGB 3'

The nucleotide sequence of wild type MGB probe (T2) is locate in the region between 1488-1503bp (the nucleotide sequence as set forth in SEQ ID NO:2) of the gene sequence associated with maternally inherited mitochondrial deafness as set forth in SEQ ID NO:5. The fluorescent reporter group is HEX fluorescent reporter group, the non-fluorescent quenching group is MGB. The formula of MGB probe is as follows:

### 5' HEX-CGTCACCCTCCTCAAG -MGB 3'

The forward primer (F1) is the nucleotide sequence between 1464-1481bp of the gene sequence associated with maternally inherited mitochondrial deafness as set forth in SEQ ID NO:5. The sequence of it corresponding to SEQ ID NO:3 in sequence list. The reverse primer (R1) is the pnucleotide sequence between 1592-1615bp, the sequence of it corresponding to SEQ ID NO:4 in sequence list.

### 3.PCR amplification reaction

The MGB probe T1, T2, forward primer F1 and reverse primer R1 were synthesized based on the designed MGB probe nucleotide sequences of SEQ ID NO:1 and SEQ ID NO:2, and the primer sequence of SEQ ID NO:3. The isolated peripheral blood DNA of said test sample was used in a real time quantitative PCR amplification reaction as template:

### Reaction system:

| | |
|---|---|
| Template | 10ng |
| Hotstar Mastermix | 10µl |
| Primer F1,R1(2pmol/1) | 1 µl each |
| MGB probe T1(2pmol/l) | 1µl |

Furthermore, if required, add 1 µl of T2(2pmol/l)

Add triple distilled water to 20µl.

### The reaction conditions were summarized in table 1:

Denaturating for 10 minutes at 95°C, then denaturating for 30 seconds at 95°C, annealing 30 seconds at 60°C, 40 circles totally. Fluorescence was detected during annealing of each cycle.

**Table 1**

| Cycle | Cycle Point |
|---|---|
| Hold @ 95°c, 10 min 0 sees | |
| Cycling (40 repeats) | Step 1 @ 95°C, hold 30 secs |
| | Step 2 @ 60 °C , hold 30 secs, acquiring to Cycling A(FAM/Sybr,JOE) |

### 4. Results

Ten of the 72 selected subjects were found carrying the C 1494T mutation in mtDNA through the detection method utilizing real time quantitative Taqman MGB probe.

The non-circled marked line in the drawings is FAM fluorescence recorded, and the circled marked line is HEX fluorescence recorded. The results of software analysis are summarized in table 2.

### 5. Validation test of the reliability of the analysis method utilizing Real time quantitative Taqman MGB probe

The 1494bp site of all individuals accepting real time quantitative Taqman MGB probe analysis were also analyzed through direct sequencing. The results suggested that there were 10 deaf patients with C1494T mutation and 62 without C1494T mutation. The results are consistent perfectly with the results from the method utilizing real time quantitative Taqman MGB probe. It confirmed the reliability and stability of the real time quantitative Taqman MGB probe method for detecting the C 1494T mutation. The data are summarized in table 2.

**Table 2**

| 72 samples | Analysis method utilizing real time quantitative fluorescence Taqman MGB probe | | Direct sequencing method |
|---|---|---|---|
| | MGB probeT1 | MGB probeT1+T2 | |
| C1494T mutant | 10, strong signal | 10 | 10 |
| C1494C wild type | 62, no or weak signal | 62 | 62 |

### Industrial applicability

The present invention relates to a TaqMan MGB probe and its related product useful for detecting the mitochondrial gene C1494T mutation associated with maternally inherited deafness. It meets the need for large scale screening of mtDNA C1494T mutation. This method is easy to operate, fast, accurate and less pollution.

## Claims

1. A real time quantitative TaqMan MGB probe useful for detecting the mitochondrial gene C1494T mutation associated with maternally inherited deafness, **characterized by** that: the 5' end of the probe is located in the region between 1482-1488 bp of the gene sequence associated with maternally inherited mitochondrial deafness as set forth in SEQ ID NO:5, the 3' end is located in the region between 1503-1508 bp of the mitochondrial gene sequence as set forth in SEQ ID NO:5, and the 5' end of the probe was labeled with fluorescent reporter group, the 3' end was labeled with non-fluorescent quenching group MGB, and the C base at position 1494bp was substituted with T, be referred to as mutant MGB probe.

2. The real time quantitative TaqMan MGB probe according to claim 1, wherein further comprising a wild type MGB probe, the wild type MGB probe is same with mutant MGB probe, except that the base at position 1494bp remaining be C, the 5' end fluorescent reporter group is different from the 5' end fluorescent reporter group of the mutant MGB probe, and the starting point of the probe is at position 1488bp rather than position 1487bp.

3. The real time quantitative TaqMan MGB probe according to claims 1 or 2, wherein the nucleotide sequence of mutant MGB probe is selected from the nucleotide sequence as set forth in SEQ ID NO:1; or the nucleotide sequence of wild type MGB probe is selected from the nucleotide sequences as set forth in SEQ ID NO:2.

4. The real time quantitative TaqMan MGB probe according to claim 3, wherein the fluorescent reporter group at the 5' end of the mutant MGB probe is FAM fluorescent reporter group, the fluorescent reporter group at the 5' end of the wild type MGB probe is HEX fluorescent reporter group.

5. A kit useful for diagnosing a maternally inherited deafness associated with mitochondrial gene mutation, the kit was used for real time quantitative detection of the C1494T mutation in the gene sequence associated with maternally inherited mitochondrial deafness as set forth in SEQ ID NO:5, so as to diagnosis maternally inherited mitochondrial deafness, comprising:
(1) Reagents useful for isolating blood DNA;
(2) Mixture of reagents useful for PCR amplification reaction;
(3) The forward primer and the reverse primer useful for amplifying the gene sequence associated with maternally inherited mitochondrial deafness as set forth in SEQ ID NO:5 comprising C1494C site or C1494T mutation site;
(4) The mutant MGB probe according to any one of claims 1 to 4;
(5) If desired, further comprising operation instruction.

6. The kit according to claim 5, wherein further comprising the wild type real time quantitative TaqMan MGB probe according to any one of claims 2 to 4.

7. The kit according to claim 5 or 6, wherein the forward primer is selected from the nucleotide sequences as set forth in SEQ ID NO:3, reverse primer is selected from the nucleotide sequence as set forth in SEQ ID NO:4.

8. The kit according to any one of claims 5 to 7, wherein the mixture of reaction reagents is Hotstar Mastermix; the forward primer and the reverse primer are mixed at a ratio of 1: 1; the reagents useful for isolating blood DNA is solution I which is used to isolate DNA from plantar blood, the principal constituents of it is Chelex.

9. The kit according to any one of claims 5 to 7, wherein the mixture of reaction reagents is Hotstar Master mix; the forward primer and the reverse primer are mixed at a ratio of 1: 1; the reagents useful for isolating blood DNA is the reagents useful for isolating DNA from peripheral blood.

10. The use of the TaqMan MGB probe according to any one of claims 1-4 or the kit according to any one of claims 5-9 in the diagnosis of the mitochondrial gene C1494T mutation associated with maternally inherited deafness in vitro.
